# EUROPÄISCHE PATENTSCHRIFT

(11) **EP 0 984 912 B1**
(45) Veröffentlichungstag und Bekanntmachung des Hinweises auf die Patenterteilung: **24.10.2001**
(21) Anmeldenummer: 98924279.7
(22) Anmeldetag: 05.05.1998
(51) Int. Cl.: C07C 37/20, C07C 37/74, C07C 37/82, C07C 39/16

(54) **VERFAHREN ZUR KONTINUIERLICHEN HERSTELLUNG VON DIHYDROXYDIPHENYLALKANEN**
METHOD FOR CONTINUOUS PRODUCTION OF DIHYDROXYDIPHENYLALKANES
PROCEDE DE PRODUCTION EN CONTINU DE DIHYDROXYDIPHENYLALCANES

(30) Priorität: 16.05.1997 DE 19720541
(43) Veröffentlichungstag der Anmeldung: 15.03.2000
(73) Patentinhaber: Bayer Aktiengesellschaft, 51368 Leverkusen (DE)
(72) Erfinder: FENNHOFF, Gerhard, D-47877 Willich (DE); BUYSCH, Hans-Josef, D-47809 Krefeld (DE); FENGLER, Gerd, D-47802 Krefeld (DE); VAN OSSELAER, Tony, D-47800 Krefeld (DE)
(86) Internationale Anmeldenummer: EP9802644
(87) Internationale Veröffentlichungsnummer: WO9852896

(56) Entgegenhaltungen:
- WO-A-92/09550
- US-A- 4 308 404
- US-A- 4 391 997
- PATENT ABSTRACTS OF JAPAN vol. 009, no. 166 (C-290), 11. Juli 1985 & JP 60 038335 A (MITSUI TOATSU KAGAKU KK), 27. Februar 1985
- DATABASE WPI Section Ch, Week 9349 Derwent Publications Ltd., London, GB; Class A41, AN 93-392597 XP002075636 & JP 05 294 873 A (CHIYODA CORP)

## Beschreibung

Vorliegende Erfindung betrifft ein Verfahren zur kontinuierlichen Herstellung von Dihydroxydiphenylalkanen (Bisphenolen), durch Umsetzung von frischem Phenol und aus der Spaltung von Beiprodukten erhaltenem Phenol mit Isoalkenylphenol und Keton. Dabei wird das Reaktionsgemisch destillativ aufgearbeitet und ein hochreines Bisphenol erzeugt. Die die Nebenprodukte enthaltenden Sümpfe und Destillate werden basisch und gegebenenfalls danach sauer gespalten. Die Spaltprodukte, die im wesentlichen aus Isoalkenylphenol und Phenol bestehen, werden gegebenenfalls nach Reinigung, mit dem während der Bisphenolreinigung erhaltenen Phenol in die Umsetzung zu Bisphenol zurückgeleitet, der aus der Spaltung erhaltene Rückstand wird entsorgt.

Es ist bekannt, Bisphenole herzustellen durch sauer katalysierte Umsetzung von Ketonen mit Phenol. Dazu gibt es eine Reihe unterschiedlicher Vorschläge (vgl. z.B. US-A 2 775 620, EP-A 342 758, EP-A 616 993, DE-OS 38 33 900, US-A 4 308 404, US-A 4308405, EP-A 630 878, US-A 4 876 391, US-A 3 242 219).

In Ullmann's Encyclopedia of Industrial Chemistry, 5. Ed., Vol. A19 p. 348-52 findet sich eine Übersicht über die ältere Literatur zur Bisphenolherstellung. In der Regel werden Bisphenole, besonders Bisphenol A (BPA), das technisch wichtigste der Bisphenole, so hergestellt, daß man Keton und Phenol zu einer im Kreis geführten Mutterlauge aus der Bishenol-Aufarbeitung hinzusetzt, dieses Gemisch über saure Ionenaustauscher leitet und die Umsetzung zu Bisphenol vollzieht. Aus dem Reaktionsgemisch wird gegebenenfalls nicht umgesetztes Keton zurückgewonnen und in die Reaktion zurückgeleitet. Das Reaktionsgemisch wird abgekühlt, das Bisphenol läßt man gegebenenfalls als Phenoladdukt auskristallisieren, trennt es ab und wäscht es mit Phenol. Aus dem Addukt wird durch Flashdestillation das Phenol abgetrennt und reines Bisphenol gewonnen. Die Mutterlauge der Kristallisation wird über saure Ionenaustauscher geleitet und darin befindliche Isomere werden zu Bisphenol umgelagert. Das so entstandene Bisphenol wird per Kristallisation abgetrennt und das erhaltene Kristallisat in den ersten Kristallisationsschritt eingeschleust. Aus der nun erhaltenen Mutterlauge zweigt man einen Teil (ca. 10 bis 20 %) ab und gibt die Hauptmenge in die Reaktion zurück. Aus dem abgezweigten Teil wird Phenol abdestilliert und in die Reaktion zurückgeführt. Den bei der Destillation erhaltenen Rückstand schleust man aus den Verfahren aus und verwendet ihn z.B. für die Herstellung von Phenolharz.

Bei dieser Verfahrensweise, die relativ kompliziert ist, wird die Mutterlauge immer wieder über den Katalysator geleitet. Dabei entstehen viele Isomere und Nebenprodukte und auch gefärbte Verbindungen, die sorgfältig von Bisphenol abgetrennt werden müssen, was gegebenenfalls eine weitere Kristallisation nötig macht. Zum anderen verliert man eine nennenswerte Menge an wertvollen Verbindungen wie Bisphenol und Isomere mit dem abgezweigten und schließlich ausgeschleusten Teil. Diese Entnahme aus der Mutterlauge ist unbedingt erforderlich, um die Menge an unbrauchbaren und störenden Verbindungen nicht zu groß werden zu lassen.

Man hat daher versucht, den ausgeschleusten Anteil aufzuarbeiten und zu spalten, um die Wertstoffe für die Bisphenolherstellung zurückzugewinnen. Auch dazu gibt es eine Reihe von Vorschlägen. Nach US-A 2 497 503 kann man durch Pyrolyse bei ca. 300°C Phenole und Alkenylphenole, die noch gründlich gereinigt werden müssen, in mäßiger Ausbeute erhalten. Auch eine hydrierende Behandlung führt zu Wertprodukten, wie EP-A 17 852 lehrt. Die Spaltung kann auch durch saure und basische Verbindungen beschleunigt werden. Mit Säuren wie Schwefel- oder Toluolsulfonsäure erhält man allerdings nur Phenol (US-A 3 466 337). Basische Katalysatoren dagegen bewirken eine Spaltung der ausgeschleusten Materialien in Phenol und Isoalkenylphenol. Als Katalysatoren werden genannt: Alkaliverbindungen wie NaOH, KOH, NaHCO₃, Na-Acetat, Na-Hypophosphit, K₂CO₃, MgO und Al-Isopropylat (US-A 4 277 628, US-A 4 594 459, US-A 4 131 749). Bei dieser Vorgehensweise wird allerdings nur ein Teil des ausgeschleusten Materials gespalten und man arbeitet diskontinuierlich oder halbkontinuierlich. Vollkontinuierliche Verfahren sind unbekannt.

Diese Verfahren sind im Hinblick auf eine höhere Reinheit des Bisphenols dahingehend verbessert worden, daß man das Phenol/Isoalkenylphenolgemisch aus der Spaltung in die erste Mutterlauge nach Abtrennung der ersten Partie Bisphenol einspeist. Man leitet das Gemisch über den sauren Katalysator und läßt die Umsetzung von Isoalkenylphenol mit Phenol und die Umlagerung gleichzeitig ablaufen. Aus der Mischung wird dann die zweite Partie Bisphenol durch Kristallisation abgetrennt und die zweite Mutterlauge wieder zur Spaltung gegeben. Die zweite Bisphenol-Partie wird in die erste Kristallisation gegeben. Dadurch wird zwar die Reinheit des Bisphenols etwas erhöht, aber man kompliziert das Verfahren um einen weiteren Kreislauf (US-A 4 954 661).

Vorgeschlagen wurde auch, die zweite Mutterlauge im oben angegebenen Bisphenolprozeß oder die erste Mutterlauge nach Abtrennen der ersten Bisphenolmenge und nach der Umlagerung zumindestens teilweise destillativ aufzuarbeiten, um die darin enthaltenen Wertprodukte besser zu nutzen. Das dabei erhaltene Bisphenol wird in die erste Kristallisationsstufe zurückgegeben und dort gereinigt. Die Isomeren enthaltenden Leichtsieder führt man der Reaktion zu (WO 94/20 445 und EP-A 552 518). Die Schwierigkeit besteht darin, aus der an Isomeren und Nebenprodukten stark angereicherten Mutterlauge mit vertretbarem Aufwand hinreichend reine Fraktionen zu erhalten, die besonderes arm an den schwer abtrennbaren Chromanen und Indanen sind, ohne daß man den nicht mehr brauchbaren Rest zu sehr vergrößert und die Ausbeute schmälert. Deswegen muß ein Teil der Destillationsprodukte verworfen werden. Die bei der Destillation erhaltenen Bisphenol-armen Fraktionen kann man auch der Spaltung unterwerfen und die Spaltprodukte in das Verfahren wieder einführen (EP-A 332 203).

Es wurde auch vorgeschlagen, Bisphenol, das aus der ersten Kristallisation im oben angegebenen Bisphenol-Verfahren stammt, durch Destillation weiter zu reinigen, wobei man ein hochreines (bis zu 99,985 %) Bisphenol erhält (EP-A 679 626).

US-A 4 391 997 offenbart eine Reaktorserie zur Herstellung von Bisphenol-A mit steigendem Temperaturgradienten.

Es wurde nun gefunden, daß man eine hervorragende Ausbeute an Bisphenol großer Reinheit nach einem einfachen vollkontinuierlichen Verfahren erhält, wenn man Phenol mit Keton und Isoalkenylphenol aus der Spaltung so umsetzt, daß möglichst wenig Isomere und Nebenprodukte entstehen, dieses Reaktionsprodukt gegebenenfalls von sauren Bestandteilen befreit, Reaktionswasser und Restaceton abdestilliert, das Reaktionsgemisch eindampft und Phenol für den Reaktionsprozeß zurückgewinnt, den erhaltenen Rückstand in einer wirksamen Destillationskolonne in ein Leichtsieder und Bisphenol enthaltendes Destillat und einen Hochsieder enthaltenden Sumpf trennt, das Destillat in einer weiteren Kolonne in ein Leichtsiederdestillat und einen hochreines Bisphenol darstellenden Sumpf trennt, das Leichtsiederdestillat mit den Hochsiedern aus der ersten Kolonne vereinigt, mit einem basischen Katalysator versetzt und kontinuierlich in einer Reaktivrektifikation spaltet, das Phenol und Isoalkenylphenol enthaltende Destillat in die Bisphenol-Reaktion leitet, den Sumpf gegebenenfalls sauer stellt und in einer weiteren Reaktivrektifikation einer zweiten Spaltung unterwirft, wobei Phenol übergeht und ein unbrauchbarer Restsumpf entsteht.

Ausgangsprodukte für das erfindungsgemäße Verfahren sind aromatische Hydroxyverbindungen und Ketone.

Geeignete aromatische Hydroxyverbindungen für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-Butylphenol, o-Cyclohexylphenol, o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenyl-phenol; besonders bevorzugt ist Phenol.

Geeignete Ketone enthalten wenigstens eine aliphtische Gruppe an der Carbonylfunktion; genannt seien beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe; besonders bevorzugt ist Aceton.

Geeignete Katalysatoren für die basische Spaltung sind die in der Literatur genannten, bevorzugt Alkalioxide und -hydroxide, besonders bevorzugt NaOH und KOH. Sie werden im erfndungsgemäßen Verfahren in die Schmelze der Spaltungsedukte eingetragen, gelöst und homogen verteilt, wobei die Temperatur zweckmäßig zwischen 100 und 200°C, bevorzugt zwischen 120 und 180°C, liegt.

Geeignete Katalysatoren für die saure Spaltung sind die in der Literatur angeführten, bevorzugt Schwefelsäure, Phosphorsäure, phosphorige Säure, deren partielle Salze wie NH₄HSO₄, NaHSO₄, KHSO₄, NaH₂PO₄, KH₂PO₄, NH₄H₂PO₄, NH₄H₂PO₃, KH₂PO₃ und Analoga, ferner die organischen Abkömmlinge dieser Säuren, nämlich aromatische Sulfonsäuren und Disulfonsäuren wie Benzol-, Toluol-, Xylol-, Phenolsulfonsäure, Diphenyldisulfonsäure, Diphenyletherdisulfonsäure, sodann aromatische Phosphon- und Phosphinsäuren wie Benzol-, Toluol-, Xylolphosphon- und -phosphinsäure, Diphenyldiphosphon- und -diphosphinsäure, ferner feste Säuren wie saure Aluminiumoxide, Tonerden wie Bentonite und Montmorillonite, Zeolithe, Titan- und Zirkonoxide, Niob- und Tantaloxide; bevorzugt sind saure Tonerden.

Die Mengen an Katalysatoren, die dem zu spaltenden Gemisch zugegeben werden, liegen zwischen 0,01 Gew.-% und 5 Gew.-%, bevorzugt zwischen 0,05 Gew.-% und 3 Gew.-%, besonders bevorzugt zwischen 0,1 Gew.-% und 2 Gew.-%, bezogen auf die Menge an Spaltgemisch. Um diese Mengenregel für saure Katalysatoren einhalten zu können, müssen die basischen Katalysatoren nach der basischen Spaltung entweder aus dem Sumpf entfernt werden, was in aller Regel zu aufwendig sein dürfte, oder sie müssen zuerst neutralisiert werden durch eine starke Säure. Dann kann der Zusatz der erforderlichen Menge an saurem Katalysator erfolgen. Auf diese Weise ist sichergestellt, daß hinreichende Mengen aktiver Säure vorliegen. Sauerstellen im Sinne der Erfindung bedeutet also, daß man dem Sumpf der basischen Spaltung soviel an Säure zugibt, daß sowohl der darin befindliche basische Katalysator neutralisiert wird, als auch darüber hinaus eine hinreichende Menge an saurem Katalysator für die folgende saure Spaltung zur Verfügung steht.

Die Katalysatoren können beispielsweise gelöst oder suspendiert in Phenol kontinuierlich dem in die Spaltkolonne fließenden Strom zudosiert werden. Es ist jedoch auch möglich, sie in Zwischenbehältern diskontinuierlich dem zu spaltenden Material beizumischen und dann dieses Gemisch kontinuierlich in die Spaltkolonne zu fördern.

Die Kolonnen, in denen die Reaktivrektifikationen durchgeführt werden, entsprechen im allgemeinen üblichen Destillationskolonnen. In den Kolonnen ist der Spaltung eine destillative und fraktionierende Trennung der Spaltprodukte, nämlich Phenol und Isoalkenylphenol, von den nichtgespaltenen oder auch nichtspaltbaren Verbindungen überlagert. Die Spaltprodukte gehen dabei, im allgemeinen schon rein genug für ihre weitere Verwendung, über Kopf. Die nicht spaltbaren Anteile werden als Sumpf ausgetragen. Reaktivrektifikationen sind dem Fachmann bekannt und beispielsweise in Ullmann's Encyclopedia of Industrial Chemistry, 5. Ed., Vol. B4, p.321-8 beschrieben.

Im vorliegenden Fall kann es vorteilhaft sein, in den Spaltkolonnen entsprechend dem während der Spaltung von oben nach unten in der Kolonnen abnehmenden Flüssigkeits- und Gasvolumen den Kolonnendurchmesser im unteren Teil angemessen abnehmen zu lassen. Für die Einstellung einer geeigneten Verweilzeit des Spaltmaterials in der Kolonne ist es zweckmäßig, Böden in die Spaltkolonnen einzuziehen. Dies gilt besonders für den Teil, in dem die Spaltung abläuft. Im oberen Teil kann für eine effektive Trennung der Produkte die Verwendung von Füllkörpern oder Packungen angezeigt sein.

Für den Fall, daß das aus der zweiten Spaltkolonne abdestillierende Phenol für die Verwendung in der Bisphenolsynthese noch nicht ausreichend rein ist, kann man es in eine weitere Kolonne oder aber in die erste Spaltkolonne einführen und dort noch weiter reinigen.

Bevorzugte Verfahrensweisen, für die Umsetzung zum Bisphenol sind solche, die möglichst hochselektiv Bisphenol liefern. Sie zeichnen sich dadurch aus, daß man bei möglichst niedriger Temperatur und möglichst niedriger Ketonkonzentration arbeitet.

Dies läßt sich erreichen, indem man Phenol mit Keton und Isoalkenylphenol in mindestens zwei in Reihe geschalteten Reaktoren umsetzt, die saure Ionenaustauscher enthalten und bei möglichst niedriger Temperatur, aber in Richtung fortschreitender Umsetzung mit steigenden Temperaturen betrieben werden, wobei die Gesamtmenge an Keton und Isoalkenylphenol auf die einzelnen Reaktoren aufgeteilt und vor dem Eintreten in die jeweiligen Reaktoren homogen im Reaktionsgemisch verteilt wird. Die Selektivität für Bisphenol kann noch erhöht werden, wenn man die Verteilung von Keton und Isoalkenylphenol so steuert, daß der Anteil der Gesamtmenge pro Reaktor umso kleiner wird, je höher die Temperatur des betreffenden Reaktors liegt und die Reaktorkaskade innerhalb einer Temperaturspanne von max. 35 bis 85°C, vorzugsweise 38 bis 75°C, betrieben wird.

Vor den einzelnen Reaktoren sind zweckmäßig sowohl Mischorgane bekannter Bauart angebracht, um die homogene Verteilung von Keton und Isalkenylphenyl im Ausgangsphenol bzw. Reaktionsgemisch zu gewährleisten als auch Wärmetauscher zur gewünschten Temperierung des Gemisches vor dem Durchgang durch das Katalysatorbett.

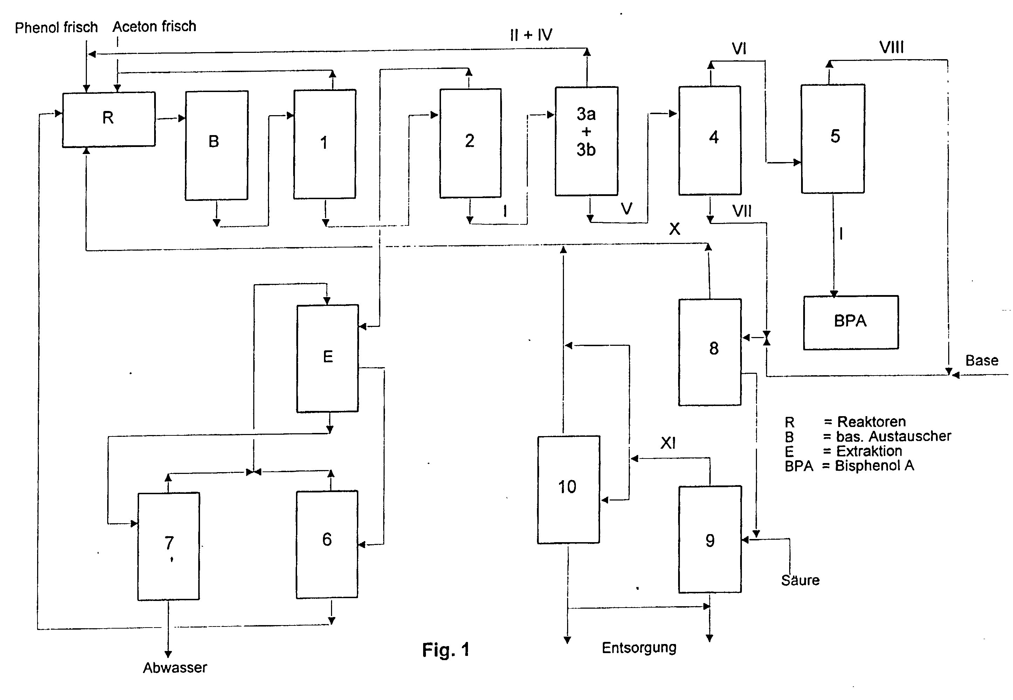

Das erfindungsgemäße Verfahren wird erläutert anhand von Abb. 1. Nach dem Verlassen des letzten Reaktors in der Kaskade, bzw. nach der Ketonabtrennung sollte das Reaktionsgemisch von sauren Bestandteilen befreit werden, wie dies beispielsweise in der EP-A 552 518 oder in der US-A 4 876 391 beschrieben ist. Gegebenenfalls sollte auch eine Feinfiltration durchgerührt werden, um Katalysator-, Apparateabrieb und sonstige feste Verunreinigungen zu entfernen. Anschließend wird aus dem Reaktionsgemisch das Reaktionswasser und nichtumgesetztes Keton abdestilliert, welch letzteres in die Reaktion zurückfließt; das Phenol enthaltende Wasser wird per Extraktion von Phenol befreit und entsorgt, der phenolhaltige Extrakt aufdestilliert, Phenol der Reaktion zugeleitet, das Extraktionsmittel wieder dem Extraktionsapparat. Diese Verfahrensweisen sind dem Fachmann bekannt.

Das nun resultierende Reaktionsgemisch wird in zwei Stufen bei vermindertem Druck zunächst von der Hauptmenge, dann von restlichem Phenol weitestgehend befreit, wobei die Sumpftemperaturen der Kolonnen etwa 250°C nicht übersteigen und die Drücke zweckmäßig <400 mbar, besonders <300 mbar, liegen sollten. Das so abdestillierte Phenol fließt in die Reaktion. Der die höher als Phenol siedenden Verbindungen enthaltende Sumpf gelangt etwa mittig in eine je nach Betriebsweise 25 bis 50 Böden repräsentierende Kolonne und wird darin in ein die Leichtsieder wie Isomere und Chromane und Bisphenol umfassendes Destillat und in einen Sumpf mit den Hochsiedern wie Indanen, Spirobisindanen, Trisphenol und restlichem Bisphenol aufgetrennt. Das Destillat wird in einer weiteren, der vorgenannten ähnlichen Kolonne mit 25 bis 50 Böden in ein Leichtsiederdestillat und einen Sumpf mit hochreinem (>99,8 %igem) Bisphenol zerlegt. Das letztere Destillat mit den Isomeren und der Sumpf aus der destillativen Abtrennung des Bisphenols enthaltend die Hochsieder und restliches Bisphenol, werden vereinigt und nach Zugabe basischer Katalysatoren vollkontinuierlich in eine Reaktivrektifikation geleitet und darin bei Sumpftemperaturen von 190 bis 270°C, bevorzugt von 200 bis 260°C, besonders bevorzugt von 210 bis 250°C, und Drücken von 15 bis 0,5 mbar, bevorzugt von 12 bis 1 mbar, besonders bevorzugt von 10 bis 1 mbar, gespalten. Das Destillat, bestehend aus Phenol und Isoalkenylphenol und dessen Oligomeren wird der Reaktion zugeleitet, der Sumpf nach Sauerstellen in eine zweite, der vorgenannten ähnlichen Reaktivrektifikation eindosiert und darin bei 150 bis 260°C, bevorzugt bei 160 bis 250°C, besonders bevorzugt bei 170 bis 240°C, und den oben angegebenen Drücken gespalten, wobei Phenol abdestilliert, das gegebenenfalls über eine Kolonne weiter gereinigt werden kann und dem Reaktionsstrom beigemischt wird und ein Sumpf am Fuß der Kolonne entnommen wird, der zu entsorgen ist.

Wenn der Sumpfanteil aus der basischen Spaltung aufgrund eines relativ kleinen Produktionsstranges einer Bisphenol-Anlage für den ökonomischen Betrieb einer Kolonne zu klein ist, empfiehlt sich eine Zusammenführung solcher Kleinmengen aus mehreren Strängen oder eine Sammlung des Sumpfes aus einer Anlage zu einer größeren Menge und dann deren Spaltung in einer geeigneten Apparatur.

Nach dem beschriebenen erfindungsgemäßen Verfahren lassen sich - nimmt man das unvermeidlich entstehende Reaktionswasser von Beginn an aus der Stoffbilanz heraus - Materialausbeuten bis zu 99 % erhalten; zudem erhält man ein besonders reines Bisphenol.

Die verwendeten Destillationseinrichtungen entsprechen dem Stand der Technik und sind dem Fachmann bekannt. In die Kolonnen zur Bisphenolabtrennung und -reinigung werden bevorzugt Füllkörper, besonders Packungen eingesetzt, die in Ullmann's Encyclopedia of Indust. Chem. 5. Ed. Vol B3, p. 4-70 bis 4-92 beschrieben sind. Auch die Spaltungskolonnen können Füllkörper und Packungen enthalten, besonders im oberen Teil, in dem die Fraktionierung erfolgt, im unteren Bereich sind jedoch Böden bevorzugt, die ebenfalls in Ullmann's Encyclopedia (l.c.) beschrieben sind.

Die die Reaktorkaskade verlassenden in die Destillation einzusetzenden Reaktionsgemische können 10 bis 35 Gew.-%, bevorzugt 12 bis 32 Gew.-%, besonders bevorzugt 14 bis 30 Gew.-%, Bisphenol enthalten. Der Gehalt an Bisphenol im von Phenol befreiten Reaktionsgemisch sollte nicht unter 90 Gew.-% betragen.

Das erfindungsgemäße Verfahren hat demnach gegenüber den bekannten Bisphenol-verfahren die Vorteile, ein hochreines Bisphenol zu liefern, mit außerordentlich großer Ausbeute bei deutlich einfacherer und vollkontinuierlicher Durchführung.

### Beispiel (vgl. Abb. 1)

In einer Kaskade von drei Reaktoren (R), die mit saurem lonenausstauscher (SC 102, Bayer AG), enthaltend 3,6 Gew.-% Dimethylthiazolidin als Cokatalysator, gefüllt waren, wurde BPA derart hergestellt, daß in den ersten Reaktor mit einer Temperatur von 55°C ca. 18 665 Gew.-Teile/h Phenol, gut vermischt mit 600 Gew.-Teilen/h Aceton und 283 Gew.-Teilen/h Destillat aus der Rückstandsspaltung, enthaltend ca. 140 Gew.-Teile Isopropenylphenol und dessen Oligomere, mit einer Menge von 0,6 kg Edukt/l Katalysator und Stunde eingeleitet wurden. Das den ersten Reaktor verlassende Produkt wurde mit 400 Gew.-Teilen/h Aceton und 187 Gew.-Teilen/h Destillat aus der Rückstandspaltung, enthaltend ca. 93 Gew.-Teilen/h Isopropenylphenol (Oligomere) gut vermischt, auf 65°C temperiert und in den zweiten Reaktor ebenfalls mit 0,6 Gew.-Teilen/l·h eingespeist. Das aus dem zweiten Reaktor fließende Produkt wurde mit 200 Gew.-Teilen/h Aceton und 94 Gew.-Teilen/h Destillat aus der Rückstandsspaltung, enthaltend ca. 46 Gew.-Teile Isopropenylphenol (Oligomere) gut vermischt, auf 75°C temperiert und in den dritten Reaktor ebenfalls mit 0,6 Gew.-Teilen/l-h eindosiert.

Der Acetonumsatz betrug dann 97 bis 98 % der Theorie. Das Reaktionsgemisch wurde über ein Bett von basischem Ionenaustauscher geleitet, um Spuren saurer Verbindungen abzufangen, und dann von Wasser und Restaceton befreit.

Das Reaktionsgemisch (20 297 Gew.-Teile/h) aus dieser Umsetzung von Phenol mit Aceton und Isopropenylphenol der in Tabelle 1 angegebenen Zusammensetzung I, das in einer ersten Destillationseinheit (1+2) schon von Reaktionswasser mit etwas Phenol und Restaceton befreit worden war (aus dem Destillat wurde Phenol durch Extraktion (E) und anschließende destillative Aufarbeitung (6,7) zurückgewonnen und wieder in die Reaktion geleitet), wurde in einer aus zwei hintereinander geschalteten Kolonnen bestehenden zweiten Destillationseinheit (3) kontinuierlich in einer ersten Kolonne (3a) von der Hauptmenge (12 476 Gew.-Teile/h, II) Phenol und das erhaltene Sumpfprodukt (7 821 Gew.-Teile/h, III) durch eine dritte Destillation in einer weiteren Kolonne (3b) weitestgehend von Phenol (3 308 Gew.-Teile/h, IV) getrennt.

Die beiden Phenoldestillate II und IV wurden in die BPA-Reaktion zurückgeführt. Der Sumpf (4 513 Gew.-Teile/h, V) wurde etwa mittig in eine Kolonne (4) mit 30 bis 40 Trennstufen eingeleitet und bei 5 mbar, ca. 215°C Kopf- und ca. 247°C Sumpftemperatur in ein BPA-reiches Destillat (4 134 Gew.-Teile/h, VI) und ein die Höhersieder enthaltendes Sumpfprodukt (302 Gew.-Teile/h, VII) aufgetrennt. In eine Kolonne (5) mit ebenfalls 30 bis 40 Trennstufen wurde das Destillat etwa mittig eingespeist und bei 5 mbar ca. 204°C Kopf- und ca. 242°C Sumpftemperatur der niedriger als BPA siedende Anteil (300 Gew.-Teile/h, VIII) abdestilliert, wobei dann im Sumpf BPA (3 824 Gew.-Teile/h, IX) hoher Reinheit erhalten wurde. Sumpfprodukt VII und Destillat VIII (602 Gew.-Teile/h) wurden kontinuierlich mit 0,2 Gew.-% KOH versetzt, etwa mittig in eine 20-bödige Kolonne (8) eindosiert und bei 3 mbar 82 bis 85°C Kopf- und 230 bis 235°C Sumpftemperatur gespalten. Das Destillat (525 Gew.Teile/h, X) floß in die Umsetzung mit Phenol zu BPA, der Sumpf (76 Gew.-Teile/h) konnte nach Zugabe von 2 mol Toluolsulfonsäure pro Mol vorhandenem KOH in einer letzten ebenfalls 20-bödigen Kolonne (9) bei etwa mittiger Eingabe bei 4 bis 5 mbar ca. 71 bis 4°C Kopf- und ca. 200°C Sumpftemperatur weiter gespalten werden, wobei ein Phenoldestillat (39 Gew.-Teile/h, XI) entstand, das (gegebenenfalls nach Reinigung in Kol. 10) ebenfalls in die BPA-Reaktion floß und ein Rückstand (37 Gew.-Teile/h) der entsorgt wurde.

Bei einer Produktion von 3 824 Gew.-Teilen/h BPA erhielt man also 37 Gew.-Teile/h Nebenprodukt entsprechend einer Materialsausbeute von >98 %.

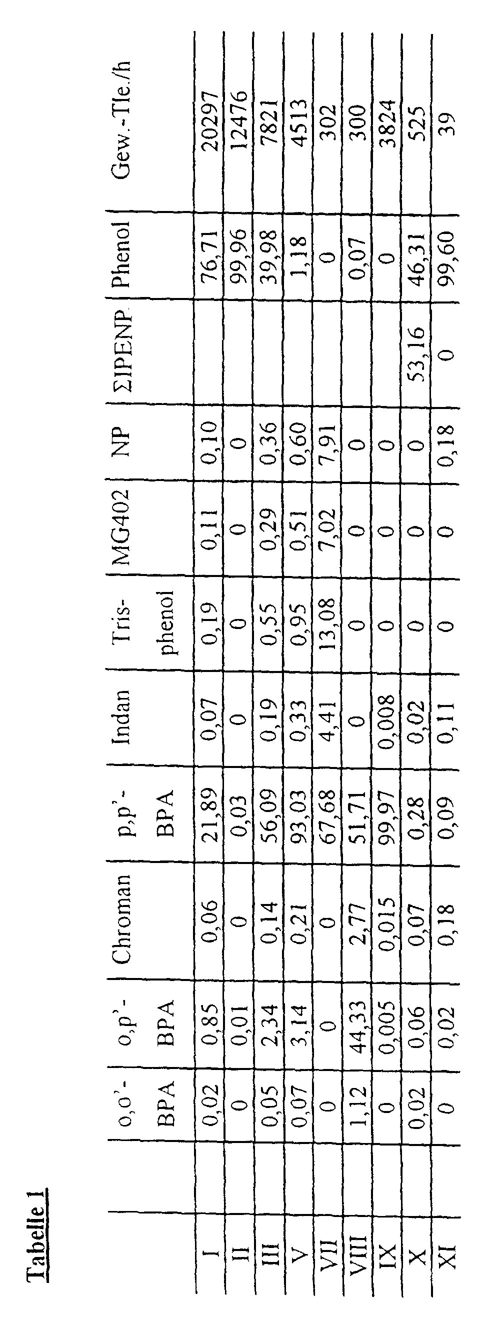

## Patentansprüche

1. Verfahren zur kontinuierlichen Herstellung von Dihydroxydiarylalkanen (Bisphenolen) aus Keton, Phenol und Isoalkenylphenol durch saure Katalyse, **dadurch gekennzeichnet, daß** man
1) in einer Kaskade von mindestens zwei Reaktoren, auf die Keton und Isoalkenylphenol aufgeteilt werden, Phenol bei in Richtung zunehmenden Umsatzes steigender Temperatur mit Keton und Isoalkenylphenol umsetzt, wobei der Anteil der Gesamtmenge Keton und Isoalkenylphenol pro Reaktor umso kleiner wird, je höher die Temperatur des betreffenden Reaktors liegt und die Reaktorkaskade innerhalb einer Temperaturspanne von 35 bis 85°C betrieben wird, und aus dem Reaktionsgemisch darin befindliche saure Bestandteile entfernt,
2) daraus Reaktionswasser und gegebenenfalls Restketon abdestilliert,
3) das trockene Reaktionsgemisch destillativ von Phenol befreit,
4) den erhaltenen, an Bisphenol hochkonzentrierten Sumpf einer weiteren Destillation unterwirft und in ein Bisphenol-reiches Destillat und einen Hochsieder enthaltenden Sumpf auftrennt,
5) dieses Destillat noch einmal destilliert, dabei die niedriger als Bisphenol siedenden Anteile über Kopf treibt und als Sumpf ein hochreines Bisphenol erhält,
6) den Hochsieder enthaltenden Sumpf aus 4) und das Leichtsieder enthaltende Destillat aus 5) vereinigt und kontinuierlich in Gegenwart einer Base in einer mehrstufigen Reaktivdestillation in über Kopf gehendes Phenol und Isoalkenylphenol und einen Hochsiedersumpf spaltet,
7) diesen Hochsiedersumpf sauer stellt und in einer weiteren Reaktivdestillation in Phenol und zu entsorgendes Harz spaltet und
8) das Phenoldestillat aus 7) und das Phenol-/Isoalkenylphenoldestillat aus 6) je gegebenenfalls nach Reinigung in die Umsetzung zu Bisphenol zurückführt.

## Claims

1. A process for the continuous production of dihydroxydiarylalkanes (bisphenols) from ketone, phenol and isoalkenylphenol by acidic catalysis, **characterised in that**
1) phenol is reacted with ketone and isoalkenylphenol, with increasing conversion with increasing temperature, in a cascade of at least two reactors into which ketone and isoalkenylphenol are apportioned, wherein the proportion of the total amount of ketone and isoalkenylphenol per reactor becomes less the higher is the temperature of the respective reactor and the reactor cascade is operated within a temperature range from 35 to 85°C, and acidic constituents contained in the reaction mixture are removed therefrom,
2) water of reaction, and possibly residual ketone, are distilled therefrom,
3) the dry reaction mixture is freed from phenol by distillation,
4) the bottom product obtained, which is highly concentrated in bisphenol, is subjected to a further distillation and is separated into a distillate rich in bisphenol and into a bottom product containing high-boiling fractions,
5) this distillate is distilled again, whereupon the fractions which are lower-boiling than bisphenol are driven overhead and a high-purity bisphenol is obtained as a bottom product,
6) the bottom product from 4), which contains high-boiling fractions, and the distillate from 5), which contains low-boiling fractions, are combined and are continuously cracked in the presence of a base, in a multi-step reactive distillation, into phenol and isoalkenylphenol which pass overhead and into a high-boiling bottom product,
7) this high-boiling bottom product is acidified and is cracked, in a further reactive distillation, into phenol and into a resin to be disposed of, and
8) the phenol distillate from 7) and the phenol-isoalkenylphenol distillate from 6) are each recycled, optionally after purification, to the reaction to form bisphenol.

## Revendications

1. Procédé pour la préparation en continu de dihydroxydiarylalcanes (bisphénols) à partir de cétone, de phénol et d'isoalcénylphénol par catalyse acide, **caractérisé en ce que**
1) dans une cascade d'au moins deux réacteurs dans lesquels on répartit la cétone et l'isoalcénylphénol, on fait réagir du phénol, à une température qui s'élève dans la direction d'une conversion croissante, avec de la cétone et de l'isoalcénylphénol, la fraction de la quantité totale de la cétone et de l'isoalcénylphénol par réacteur étant d'autant plus faible que la température du réacteur concerné est élevée et la cascade de réacteurs étant mise en service dans les limites d'une plage de température de 35 à 85°C, et on élimine du mélange réactionnel les constituants acides qui s'y trouvent,
2) on sépare par distillation l'eau réactionnelle et, le cas échéant, la cétone résiduelle,
3) on libère du phénol par distillation le mélange réactionnel séché,
4) on soumet à une distillation supplémentaire le produit de bas de colonne obtenu possédant une concentration élevée en bisphénol et on le sépare en un produit de distillation riche en bisphénol et en un produit de bas de colonne contenant des composants à point d'ébullition élevé,
5) on distille encore une fois ce produit de distillation; en l'occurrence, on entraîne, via la tête de colonne, les fractions dont le point d'ébullition est inférieur à celui du bisphénol et on obtient, à titre de produit de bas de colonne, du bisphénol très pur,
6) on combine le produit de bas de colonne contenant des composants à point d'ébullition élevé provenant de 4) et le produit de distillation contenant des composants à bas point d'ébullition provenant de 5) et on les coupe en continu, en présence d'une base, sous la forme d'une distillation par réaction en plusieurs étapes, en phénol et en isoalcénylphénol qui s'évacuent via la tête de colonne et en un produit de bas de colonne contenant des composants à point d'ébullition élevé,
7) on acidifie ce produit de bas de colonne contenant des composants à point d'ébullition élevé et, au cours d'une distillation supplémentaire par réaction, on le coupe en phénol et en résine à éliminer, et
8) on recycle, dans la mise en réaction pour obtenir du bisphénol, le produit de distillation de phénol provenant de 7) et le produit de distillation de phénol/isoalcénylphénol provenant de 6), le cas échéant, après les avoir soumis respectivement à une purification.
